# EUROPÄISCHE PATENTSCHRIFT

(11) **EP 1 475 119 B1**
(45) Veröffentlichungstag und Bekanntmachung des Hinweises auf die Patenterteilung: **17.05.2006**
(21) Anmeldenummer: 04003926.5
(22) Anmeldetag: 20.02.2004
(51) Int. Cl.: A61M 16/20

(54) **Vorrichtung zur Steuerung einer Gasströmung**
Device for controlling gas flow
Appareil de régulation d'un écoulement de gaz

(30) Priorität: 08.05.2003 DE 10320454
(43) Veröffentlichungstag der Anmeldung: 10.11.2004
(73) Patentinhaber: Weinmann Geräte für Medizin GmbH & Co. KG, 22525 Hamburg (DE)
(72) Erfinder: Paesch, Rainer, 25451 Quickborn (DE); Schulte, André, 22765 Hamburg (DE)
(74) Vertreter: Klickow, Hans-Henning

(56) Entgegenhaltungen:
- EP-A- 0 806 216
- WO-A-01/41856
- WO-A-99/22795
- DE-A- 10 131 653
- US-A1- 2002 073 998
- MURRAY T M; KRUSE M L; BATTCHER J A; WOODS J W; EDMONDS H L; PALAHEIMO M P: ""Hands-Off"-Voice Activated Automated Anesthesia Recordkeeping And Monitoring System (ARMS)" PROCEEDINGS - 1990 SOUTHEASTON, Bd. 3, 8. Januar 1991 (1991-01-08), Seiten 822-824, XP000202499

## Beschreibung

Die Erfindung betrifft eine Vorrichtung zur Steuerung einer Gasströmung, die mindestens ein pneumatisch steuerbares Verstärkerventil aufweist, das gemeinsam mit einer Dosiereinrichtung in einem Ventilblock angeordnet ist

Derartige Verstärkerventile werden in Kombination mit Dosiereinrichtungen beispielsweise bei Beatmungsgeräten eingesetzt, um das Strömungsvolumen eines Atemgases vorzugeben. Die Verstärkerventile werden häufig in Kombination mit separaten Dosiervorrichtungen und Stelleinrichtungen betrieben.

Aus der WO 99/22795 A ist bereits eine Vorrichtung zur Steuerung einer Gasströmung bekannt, die ein pneumatisch steuerbares Verstärkerventil aufweist, das gemeinsam mit einer Dosiereinrichtung in einem Ventilblock angeordnet ist. Die Dosiereinrichtung weist einen Kolben mit einem Kolbenschaft auf. Der Kolbenschaft ist in einem Endbereich mit einem Pfropfen aus Kunststoff versehen, der zur Abdichtung einer Ausströmdüse bzw. zur Vorgabe eines Strömungswiderstandes vorgesehen ist.

Aus dem Zeitschriftenaufsatz MURRAY TM; KRUSE M L; BATTCHER J A; WOOODS J W; EDMONDS H L; PALAHEIMO M P: "Hands-Off'Voice Activated Automated Anesthesia Recordkeeping and Monitoring System (ARMS)' PROCEEDINGS - 1990 SOUTHEASTON, Bd. 3, 8. Januar 1991 (1991-01-08), Seiten 822 -824, ist es bekannt, ein Sprachmodul für ein Narkoseüberwachungs- und Aufzeichnungssystem zu verwenden.

Aus der WO 01/41856 A ist es bekannt, bei einer Gasversorgungseinrichtung ein akustisches Alarmsignal bei Erreichen vorgegebener Betriebsbedingungen zu generieren.

In der DE 101 31 653 A wird eine Einrichtung zur Atemgasversorgung einer Atemmaske beschrieben, bei der ein Ausatemventil zur Unterstützung einer Auswaschung von Kohlendioxid angesteuert wird.

Aufgabe der vorliegenden Erfindung ist es, eine Vorrichtung der einleitend genannten Art derart zu konstruieren, daß bei mindestens gleicher Funktionsqualität eine kompaktere Ausführungsform bereitgestellt wird.

Diese Aufgabe wird erfindungsgemäß dadurch gelöst, daß im Bereich des Ventilblockes ein Druckregler angeordnet ist, daß die Dosiereinrichtung mit einem in einer Kanalerweiterung eines Dosierkanals angeordneten Dosierelement versehen ist und daß ein in Richtung auf den Dosierkanal positionierbarer Primärkolben der Dosiereinrichtung mit einem Abstand zum Dosierelement positionierbar ist.

Durch die Anordnung sowohl des Verstärkerventils als auch der Dosiereinrichtung in einem gemeinsamen Ventilblock wird eine Funktionseinheit bereitgestellt, die nur sehr wenig Platz benötigt. Eine derartige Vorrichtung ist deshalb insbesondere für eine Verwendung bei sehr kleinen Beatmungsgeräten geeignet. Typischer Weise wird die Dosiereinrichtung mit einem Dosierventil versehen.

Zur Unterstützung einer vollständigen Unterbindung der Gasströmung ist vorgesehen, daß über das Verstärkerventil eine Ausschaltfunktion realisierbar ist.

Eine sehr präzise und reproduzierbare Dosierung wird dadurch unterstützt, daß die Dosiereinrichtung für eine hysteresefreie Dosierung ausgebildet ist.

Zur Erleichterung einer Gerätebedienung ist vorgesehen, daß eine Bedieneinheit mit einem Sprachmodul versehen ist.

Eine Erkennung von Fehlfunktionen wird dadurch unterstützt, daß die Bedieneinheit einen Signalgenerator für ein akustisches Alarmsignal aufweist.

Zur Unterstützung eines individuellen anpaßbaren Steuerungsverhaltens wird vorgeschlagen, daß zur Ansteuerung des Verstärkerventils ein pneumatisches Zeitglied verwendet ist. Alternativ kann auch ein elektro-pneumatisches Zeitglied eingesetzt werden.

Eine konstruktiv einfache Realisierung der Dosierung besteht darin, daß die Dosiereinrichtung mit einem in einer Kanalerweiterung angeordneten Dosierelement versehen ist.

Eine sehr präzise geometrische Formung im Bereich der Dosiereinrichtung wird dadurch unterstützt, daß das Dosierelement kugelartig ausgebildet ist.

Gemäß einer anderen Ausführungsform ist auch daran gedacht, daß das Dosierelement kegelartig ausgebildet ist.

Eine präzise Dosierung wird dadurch unterstützt, daß die Dosiereinrichtung ein Dosierventil mit einem Primärkolben aufweist, der mit einem Anschlagelement für das Dosierelement versehen ist.

Zur Erreichung eines sehr gleichmäßigen Steuerungsverhaltens wird vorgeschlagen, daß im Bereich des Ventilblockes ein Druckregler angeordnet ist.

Eine weitere Realisierung der Dosiereinrichtung besteht darin, daß die Dosiereinrichtung elektromechanisch gesteuert ausgebildet ist.

Eine Einstellung der Dosiereinrichtung kann beispielsweise dadurch erfolgen, daß die elektromechanische Steuerung als ein Motor ausgebildet ist.

Eine sehr kompakte Konstruktion kann dadurch erreicht werden, daß die elektromechanische Steuerung als ein Piezoantrieb ausgebildet ist.

Ein typisches Anwendungsgebiet besteht darin, daß die Dosiereinrichtung für eine Anwendung im Bereich der Beatmungstechnik ausgebildet ist.

Ebenfalls ist hinsichtlich einer bevorzugten Anwendung daran gedacht, daß der Ventilblock zum Anschluß an eine Druckgasflasche zur Bevorratung von Sauerstoff ausgebildet ist.

In den Zeichnungen sind Ausführungsbeispiele der Erfindung schematisch dargestellt. Es zeigen:
- Fig. 1: ein schematisches Blockschaltbild zur Veranschaulichung der wesentlichen Funktionskomponenten,
- Fig. 2: einen schematischen Querschnitt durch einen Ventilblock mit Verstärkerventil und Dosiereinrichtung,
- Fig. 3: eine vergrößerte Darstellung des ventilblockes gemäß Figur 2 im Bereich der Dosiereinrichtung und
- Fig. 4: eine Darstellung der Dosiereinrichtung gemäß Figur 3 in einem anderen Betriebszustand und mit einem anders positionierten Kolben des Verstärkerventils.

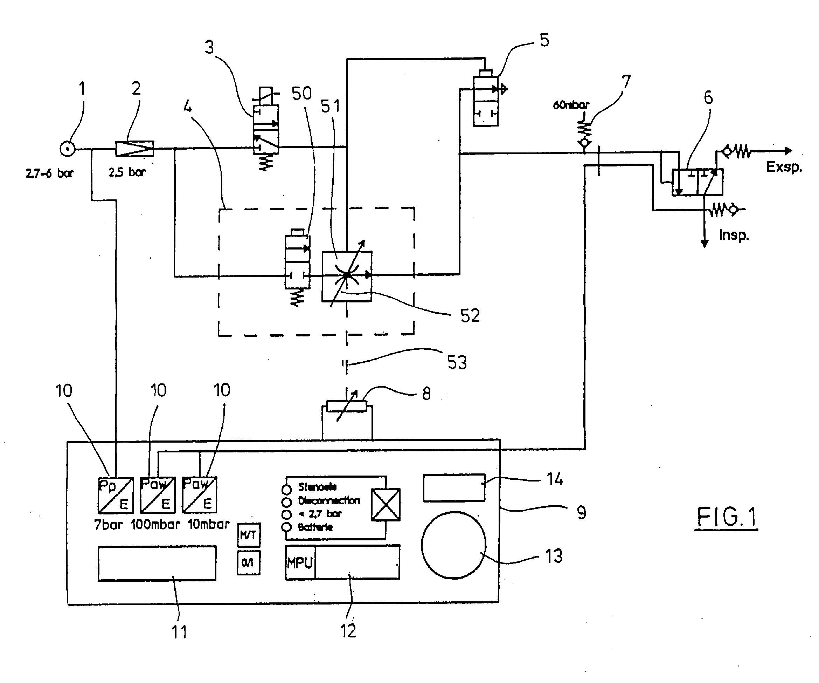

Figur 1 zeigt einen Übersichtsplan der wesentlichen Funktionskomponenten eines Beatmungsgerätes, das mit der Vorrichtung zur Steuerung der Gasströmung sowie einer Bedieneinheit versehen ist von einer Gasversorgung (1) wird das Gas, beispielsweise das Atemgas, bereitgestellt. Ein typischer Druck im Bereich der Gasversorgung (1) liegt im Bereich von 2,7 bis 6 bar. Von der Gasversorgung (1) wird das Gas zunächst einem Druckregler (2) zugeführt, der einen Basisdruck bereitstellt. Im Ausführungsbeispiel liefert der Druckregler (2) einen Druck von etwa 2,5 bar.

Der Druckregler (2) ist mit einem als 3/2-Wegeventil ausgebildetem Pilotventil (3) und einer Dosiereinrichtung (4) verbunden. Das Pilotventil (3) und die Dosiereinrichtung (4) sind mit einem Entlüftungsventil (5) verbunden. Darüber hinaus erfolgt ein Anschluß an ein Patientenventil (6), das bei Anwendungen im Bereich der Beatmungstechnik eine Funktion in Abhängigkeit von Inspirationsphasen und Expirationsphasen ausübt. Im verbindungbereich zwischen der Dosiereinrichtung (4) und dem Patientenventil (6) ist ein Überdruckventil (7) angeordnet, das den hier herrschenden Druck auf maximal 60 mbar begrenzt.

Eine Einstellspindel der Dosiereinrichtung (4) ist über ein Kupplungsglied (53) mit einem als ein Potentiometer ausgebildeten Stellelement (8) verbunden, das bei Anwendungen im Bereich der Beatmungstechnik in Abhängigkeit von einem jeweiligen Atemvolumen einen Sollwert für die Atemfrequenz vorgibt. Das Stellelement (8) ist darüber hinaus an eine Bedieneinheit (9) angeschlossen. Im Bereich der Bedieneinheit (9) sind Druckwandler (10) sowie eine Druckanzeige (11) angeordnet. Die Druckwandler (10) transformieren einen jeweils gemessenen Druck in ein elektrisches Meßsignal.

Zur Unterstützung einer Bedienerführung ist ein Sprachmodul (12) verwendet. Das Sprachmodul (12) unterstützt einen jeweiligen Bediener und gibt die Sprachsignale über einen Schallgeber (13) aus. Zur Unterstützung einer netzunabhängigen Versorgung ist eine Batterie (14) verwendet.

Aus Figur 1 ist ebenfalls zu erkennen, daß die Dosiereinrichtung (4) mit einem Verstärkerventil (50) und einem Dosierventil (51) versehen ist. Das Dosierventil (51) weist eine Stellachse (52) auf. Das Verstärkerventil (50) ist in Strömungsrichtung zwischen dem Druckregler (2) und dem Dosierventil (51) angeordnet. Über das Dosierventil (51) erfolgt der Anschluß an das Entlüftungsventil (5) sowie das Patientenventil (6).

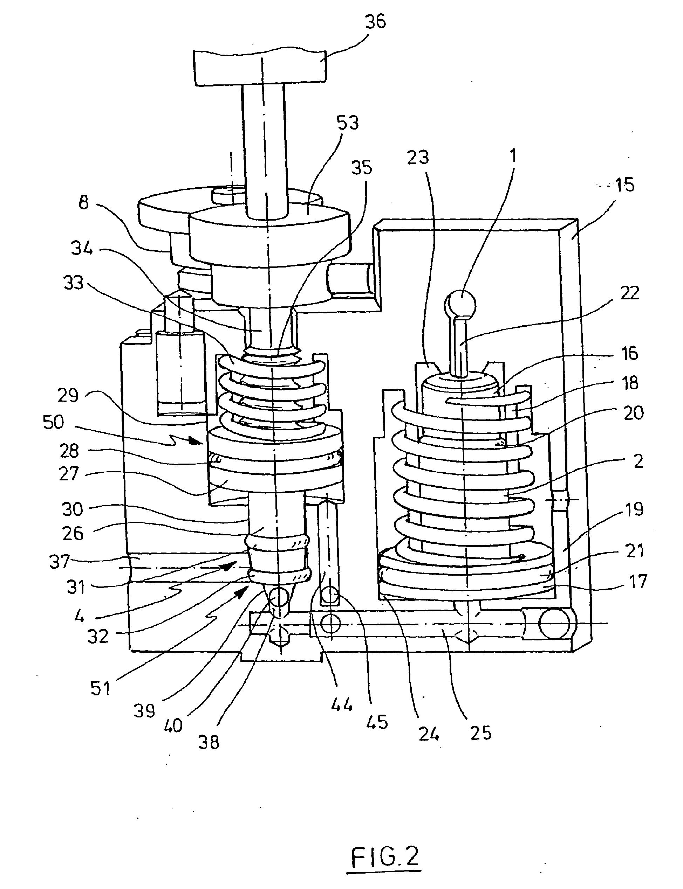

Figur 2 zeigt einen Ventilblock (15), in dessen Bereich der Druckregler (2), ein Anschlußkanal (45) zum nicht dargestellten Pilotventil (3) sowie die Dosiereinrichtung (4) angeordnet sind. Aus Figur 2 ist erkennbar, daß der Druckregler (2) aus einem Primärkolben (16) und einem Sekundärkolben (17) besteht, die starr miteinander verbunden sind. Der Sekundärkolben (17) hat einen größeren Durchmesser als der Primärkolben (16).

Gegenüber von Führungswandungen (18, 19) sind die Kolben (16, 17) durch Dichtringe (20, 21) abgedichtet. Die Gasversorgung (1) ist an einen Zuführkanal (22) angeschlossen, der in einen Arbeitsraum (23) des Primärkolbens (16) einmündet. Ein Arbeitsraum (24) des Sekundärkolbens (17) ist mit einem Querkanal (25) des ventilblockes (15) verbunden.

Die Dosiereinrichtung (4) besteht im wesentlichen aus dem Verstärkerventil (50) und dem Dosierventil (51). Das Verstärkerventil (50) besteht aus einem Primärkolben (26) und einem Sekundärkolben (27), wobei der Sekundärkolben (27) einen größeren Durchmesser als der Primärkolben (26) aufweist. Der Sekundärkolben (27) ist von einer Dichtung (28) gegenüber einer Führungswandung (29) abgedichtet. Der Primärkolben (26) weist zur Abdichtung gegenüber der ihm zugeordneten Führungswandung (30) zwei Dichtungen (31, 32) auf. Das Verstärkerventil (50) ist als ein 2/2-Wege-Ventil ausgebildet.

Auch bei der Dosiereinrichtung (4) mit Verstärkerventil (50) sind der Primärkolben (26) und der Sekundärkolben (27) starr miteinander verbunden. Im Bereich seiner dem Primärkolben (26) abgewandten Ausdehnung wird der Sekundärkolben (27) von einer Druckfeder (33) beaufschlagt. Der vorhandene Federdruck ist so groß, daß der Primärkolben (26) mit dem Dichtring (32) die Öffnung der Dosiereinrichtung (4) verschließt. Hierdurch wird die Funktion des Dosierventils (51) ausgeübt.

Nach einer Druckbeaufschlagung durch das Pilotventil (3) über den Anschlußkanal (45) und den Steuerkanal (44) auf die Unterseite des Sekundärkolbens (27) gleitet der Kolben gegen die Kraft der Druckfeder (33) nach oben gegen die Einstellspindel (35). Die Einstellspindel ist über einen Schaft (34) mit einem Einstellelement (36) verbunden.

Der Schaft (34) trägt ein Zahnrad, das einen Teil des Kopplungsgliedes (53) ausbildet. Der andere Teil des Kopplungsgliedes wird von einem weiteren Zahnrad gebildet, das in das erste Zahnrad eingreift. Das zweite Zahnrad ist mit dem als Potentiometer ausgebildeten Stellglied (8) verbunden. Bei einer Rotation des Schaftes (34) wird hierdurch eine Verstellung des Potentiometers hervorgerufen.

In Abhängigkeit von einer jeweiligen Positionierung des Dosierventils (51) durch die Einstellspindel (35) wird ein Ausgangskanal (37) zu einem vorgebbaren Anteil verschlossen. Der jeweilige Verschlußzustand gibt die Strömungsmenge der Gasströmung vor. Der Ausgangskanal (37) leitet die Gasströmung in Richtung auf das Patientenventil (6).

Der Querkanal (25) mündet im Bereich seiner dem Druckregler (2) abgewandten Ausdehnung in einen Dosierkanal (38) ein. Beim dargestellten Ausführungsbeispiel erweitert sich der Dosierkanal (38) trichterförmig in Richtung auf den Primärkolben (26). In der hierdurch gebildeten Kanalerweiterung (39) ist ein Dosierelement (40) geführt, das beim dargestellten Ausführungsbeispiel als eine Kugel ausgebildet ist.

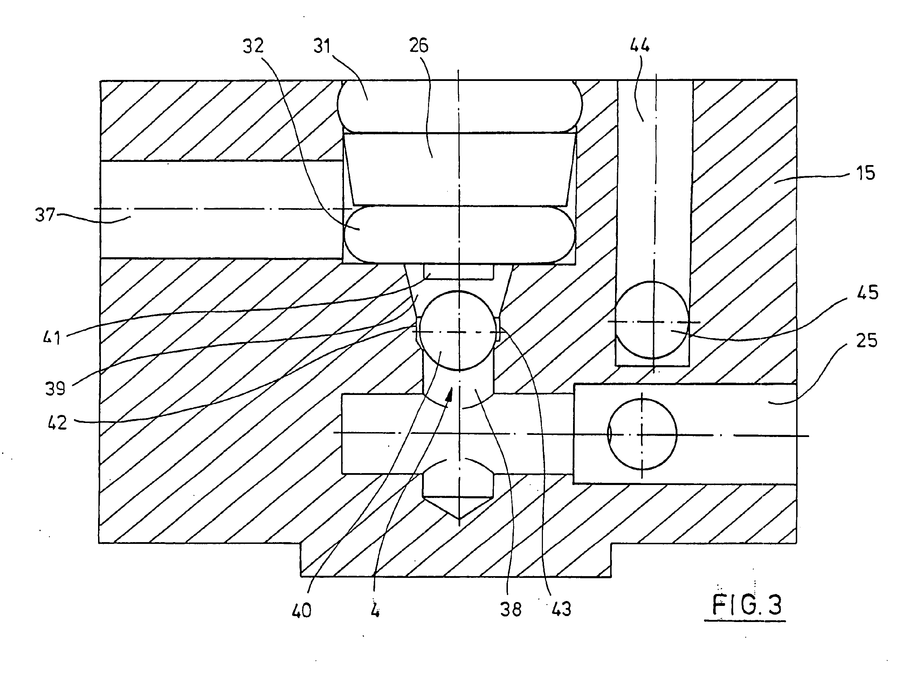

Figur 3 veranschaulicht die Gestaltung des Dosierkanals (38) mit dem Dosierelement (40) sowie die Positionierung des Primärkolbens (26) in einer gegenüber Figur 2 vergrößerten Darstellung. Der Primärkolben (26) ist bei der dargestellten Positionierung soweit in Richtung auf den Dosierkanal (38) verschoben, daß die Dichtung (32) den Dosierkanal (38) verschließt. Es kann hierdurch kein Gas vom Dosierkanal (38) in den Ausgangskanal (37) übertreten. Ein Anschlagelement (41) des Primärkolbens (26) ist beim dargestellten Betriebszustand mit einem Abstand zum Dosierelement (40) positioniert. Zwischen dem Dosierelement (40) und einer Kanalwandung (42) des Dosierkanals (38) erstreckt sich ein Ringspalt (43) für eine Minimaldosierung. Die Unterseite des Sekundärkolbens (27) wird von dem kleinen Pilotventil (3) mit Druck beaufschlagt und gegen die Eistellspindel (35) geschoben. Die Druckzuleitung erfolgt durch den Steuerkanal (44) hindurch.

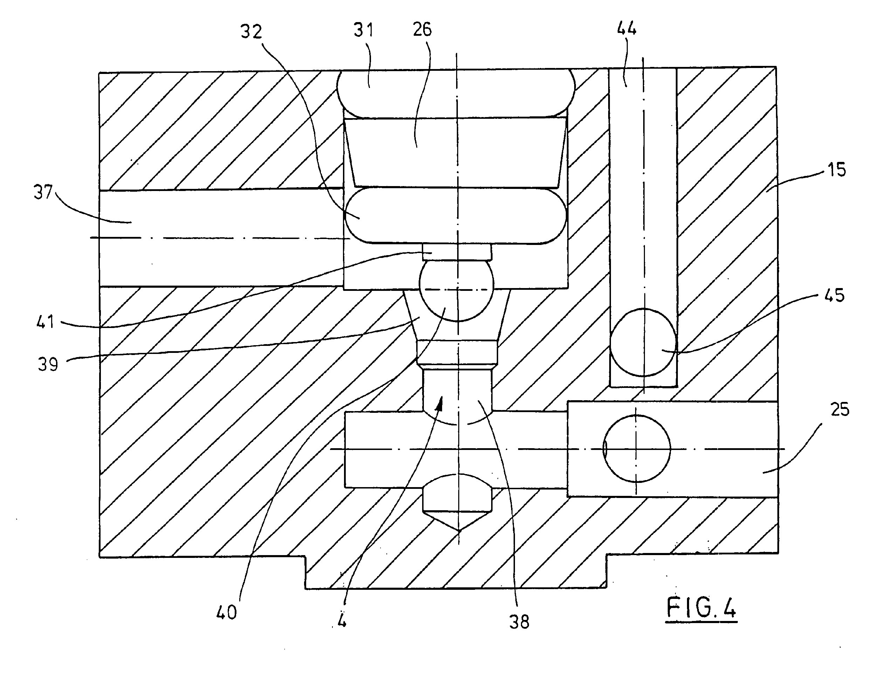

Bei der in Figur 4 dargestellten Positionierung des Dosierventils (51) ist die Dichtung (32) mit einem Abstand zum Dosierkanal (38) angeordnet. Hierdurch kann Gas aus dem Dosierkanal (38) in Richtung auf den Ausgangskanal (37) strömen. Durch die Gasströmung wird das Dosierelement (40) gegen das Anschlagelement (41) gedrückt. Der Ringspalt im Bereich der Kanalerweiterung (39) um das Dosierelement (40) herum bestimmt den jeweiligen Durchfluß. Zu erkennen ist auch, daß das Anschlagelement (41) eine Mulde aufweist, deren Kontur an die Oberflächenkrümmung des Dosierelementes (40) angepaßt ist. Verformungen des Dosierelementes (40) werden hierdurch vermieden.

Bei einer Abschaltung über das Pilotventil (3) wird der Raum unterhalb der Fläche des Sekundärkolbens (27) entlüftet und die Druckfeder (33) schiebt den Sekundärkolben (27) und damit den Primärkolben (26) in Richtung auf den Dosierkanal (38), so daß die Dichtung (32) den Dosierkanal (38) wieder verschließt.

## Patentansprüche

1. Vorrichtung zur Steuerung einer Gasströmung, die mindestens ein pneumatisch steuerbares Verstärkerventil (50) aufweist, das gemeinsam mit einer Dosiereinrichtung (4) in einem Ventilblock (15) angeordnet ist, **dadurch gekennzeichnet, daß** im Bereich des Ventilblockes (15) ein Druckregler (2) angeordnet ist, daß die Dosiereinrichtung (4) mit einem in einer Kanalerweiterung (39) eines Dosierkanals (38) angeordneten Dosierelement (40) versehen ist und daß ein in Richtung auf den Dosierkanal (38) positionierbarer Primärkolben (26) der Dosiereinrichtung (4) mit einem Abstand zum Dosierelement (40) positionierbar ist.

2. Vorrichtung nach Anspruch 1, **dadurch gekennzeichnet, daß** über das Verstärkerventil (50) eine Ausschaltfunktion realisierbar ist.

3. Vorrichtung nach einem der Ansprüche 1 bis 2, **dadurch gekennzeichnet, daß** eine Bedieneinheit (9) mit einem Sprachmodul (12) versehen ist.

4. Vorrichtung nach einem der Ansprüche 1 bis 3, **dadurch gekennzeichnet, daß** die Bedieneinheit (9) einen Signalgenerator für ein akustisches Alarmsignal aufweist.

5. Vorrichtung nach einem der Ansprüche 1 bis 4, **dadurch gekennzeichnet, daß** zur Ansteuerung des Verstärkerventils (50) ein pneumatisches Zeitglied verwendet ist.

6. Vorrichtung nach einem der Ansprüche 1 bis 5, **dadurch gekennzeichnet, daß** das Dosierelement (40) kugelartig ausgebildet ist.

7. Vorrichtung nach einem der Ansprüche 1 bis 5, **dadurch gekennzeichnet, daß** das Dosierelement (40) kegelartig ausgebildet ist.

8. Vorrichtung nach einem der Ansprüche 1 bis 7, **dadurch gekennzeichnet, daß** die Dosiereinrichtung (4) ein Dosierelement (51) mit einem Primärkolben (26) aufweist, der mit einem Anschlagelement (41) für das Dosierelement (40) versehen ist.

9. Vorrichtung nach einem der Ansprüche 1 bis 8, **dadurch gekennzeichnet, daß** die Dosiereinrichtung (4) elektromechanisch gesteuert ausgebildet ist.

10. Vorrichtung nach Anspruch 9, **dadurch gekennzeichnet, daß** die elektromechanische Steuerung als ein Motor ausgebildet ist.

11. Vorrichtung nach Anspruch 9, **dadurch gekennzeichnet, daß** die elektromechanische Steuerung als ein Piezoantrieb ausgebildet ist.

12. Vorrichtung nach einem der Ansprüche 1 bis 11, **dadurch gekennzeichnet, daß** die Dosiereinrichtung (4) für eine Anwendung im Bereich der Beatmungstechnik ausgebildet ist.

13. Vorrichtung nach einem der Ansprüche 1 bis 12, **dadurch gekennzeichnet, daß** der Ventilblock (15) zum Anschluß an eine Druckgasflasche zur Bevorratung von Sauerstoff ausgebildet ist.

## Claims

1. Device to control a gas flow, said device having at least one pneumatically controllable amplifier valve (50), which is arranged together with a metering device (4) in a valve block (15), **characterized in that** in the region of the valve block (15) a pressure regulator (2) is arranged, that the metering device (4) has a metering element (40) which is arranged in a channel extension (39) of a metering channel (38), and that a primary piston (26) - which can be positioned in the direction towards the metering channel (38) - of the metering device (4) can be positioned at a distance from the metering element (40).

2. Device according to Claim 1, **characterized in that** a switching-off function can be implemented via the amplifier valve (50).

3. Device according to one of Claims 1 to 2, **characterized in that** an operating unit (9) with a speech module (12) is provided.

4. Device according to one of Claims 1 to 3, **characterized in that** the operating unit (9) has a signal generator for an acoustic alarm signal.

5. Device according to one of Claims 1 to 4, **characterized in that** to control the amplifier valve (50), a pneumatic timing element is used.

6. Device according to one of Claims 1 to 5, **characterized in that** the metering element (40) is spherical.

7. Device according to one of Claims 1 to 5, **characterized in that** the metering element (40) is conical.

8. Device according to one of Claims 1 to 7, **characterized in that** the metering device (4) has a metering element (51) with a primary piston (26), which has a limit stop element (41) for the metering element (40).

9. Device according to one of Claims 1 to 8, **characterized in that** the metering device (4) is in a form which is controlled electromechanically.

10. Device according to Claim 9, **characterized in that** the electromechanical control is in the form of a motor.

11. Device according to Claim 9, **characterized in that** the electromechanical control is in the form of a piezo drive.

12. Device according to one of Claims 1 to 11, **characterized in that** the metering device (4) is in a form for use in the field of respiration technology.

13. Device according to one of Claims 1 to 12, **characterized in that** the valve block (15) is in a form for connection to a compressed gas cylinder for supplying oxygen.

## Revendications

1. Dispositif pour la commande d'un flux de gaz présentant au moins une soupape amplificatrice pneumatique (50) qui est disposée, en commun avec un dispositif de dosage (4), dans un bloc de soupapes (15), **caractérisé en ce que**, dans la région du bloc de soupapes (15), est disposé un régulateur de pression (2), que le dispositif de dosage (4) est pourvu d'un élément de dosage (40), disposé dans un élargissement (39) d'un canal de dosage (38), et qu'un piston principal (26) du dispositif de dosage (4), positionnable dans une direction pointée sur le canal de dosage (38), peut être positionné à une distance de l'élément de dosage (40).

2. Dispositif selon la revendication 1, **caractérisé en ce que**, par l'intermédiaire de la soupape amplificatrice (50), une fonction de mise hors circuit peut être réalisée.

3. Dispositif selon l'une des revendications 1 à 2, **caractérisé en ce que** l'unité de commande (9) est pourvue d'un module vocal (12).

4. Dispositif selon l'une des revendications 1 à 3, **caractérisé en ce que** l'unité de commande (9) présente un générateur de signaux pour un signal d'alarme acoustique.

5. Dispositif selon l'une des revendications 1 à 4, **caractérisé en ce que**, pour l'excitation de la soupape amplificatrice (50), on utilise un organe de temporisation pneumatique.

6. Dispositif selon l'une des revendications 1 à 5, **caractérisé en ce que** l'élément de dosage (40) est en forme de boule.

7. Dispositif selon l'une des revendications 1 à 5, **caractérisé en ce que** l'élément de dosage (40) est conçu en forme de cône.

8. Dispositif selon l'une des revendications 1 à 7,
**caractérisé en ce que** le dispositif de dosage (4) présente un élément de dosage (51) avec un piston principal (26) qui est pourvu d'un élément de butée (41) pour l'élément de dosage (40).

9. Dispositif selon l'une des revendications 1 à 8, **caractérisé en ce que** le dispositif de dosage (4) est à commande électromécanique.

10. Dispositif selon la revendication 9, **caractérisé en ce que** la commande électromécanique consisté en un moteur.

11. Dispositif selon la revendication 9, **caractérisé en ce que** la commande électromécanique est conçu en tant que commande piézoélectrique.

12. Dispositif selon l'une des revendications 1 à 11, **caractérisé en ce que** le dispositif de dosage (4) est conçu pour l'utilisation dans le domaine de la technique de la respiration artificielle.

13. Dispositif selon l'une des revendications 1 à 12, **caractérisé en ce que** le bloc de soupapes (15) est conçu pour le raccordement à une bouteille à gaz comprimé pour le stockage d'oxygène.
